Europäisches Patentamt

European Patent Office

Office européen des brevets

(11) Numéro de publication: **0 340 056**

A1

# (12) DEMANDE DE BREVET EUROPEEN

(21) Numéro de dépôt: 89400892.9

(22) Date de dépôt: 31.03.89

(51) Int. Cl.⁴: **C 07 K 1/14**
C 07 K 5/00, C 07 K 5/06

(30) Priorité: 06.04.88 FR 8804516

(43) Date de publication de la demande:
02.11.89 Bulletin 89/44

(84) Etats contractants désignés:
AT BE CH DE ES FR GB GR IT LI LU NL SE

(71) Demandeur: RHONE-POULENC CHIMIE
25, quai Paul Doumer
F-92408 Courbevoie Cédex (FR)

(72) Inventeur: Bres, Hervé
Im. La Clairière 42, rue du Commandant Charcot
F-69005 - Lyon (FR)

Gervais, Christian
6, allée Marcel Achard
F-69100 - Villeurbanne (FR)

Casati, Jean-Pierre
43, rue Pierre Sémard
F-69520 - Grigny (FR)

(74) Mandataire: Le Pennec, Magali et al
Rhone-Poulenc Chimie Service Brevets Chimie 25 Quai
Paul Doumer
F-92408 Courbevoie (FR)

(54) Procédé de purification de peptides.

(57) La présente invention concerne un procédé de purification et d'extraction du dipeptide ε trifluoroacétyl-lysyl-proline à partir de solution aqueuse saturée en sel contenant le dipeptide, des polypeptides et des acides aminés isolés par une série d'extractions successives par un alcool primaire ou un mélange alcool primaire solvant-aprotique.

EP 0 340 056 A1

## Description

## PROCEDE DE PURIFICATION DE PEPTIDES

La présente invention concerne un procédé de purification du dipeptide ε trifluoroacétyl-lysyl-proline à partir des solutions aqueuses obtenues lors de sa préparation, elle concerne plus particulièrement la purification de l'ε trifluoroacétyl-L lysyl-L -proline.

Le dipeptide est une matière première utilisée dans la synthèse d'un agent hypertenseur décrit dans le brevet européen n° 168 769. Ce dérivé ayant une utilisation pharmaceutique doit se présenter sous une forme extrêmement pure exempte de composés polypeptides contenant plus de deux acides aminés, d'aminoacides libres et de sels.

Le procédé de préparation du dipeptide ε trifluoroacétyl-lysyl-proline requiert plusieurs étapes. Par exemple dans une première étape à partir de la lysine on protège le groupe amine situé en position ε régiosélectivement. La protection de la fonction ε régiosélective se fait grace à l'utilisation d'esters de l'acide trifluoroacétique telle que décrit par exemple dans la demande non encore publiée déposée en Europe sous le n° 88-400088.6. Dans cette demande est décrit un procédé de N-ω trifluoroacétylation des α, ω diaminoacides par un trifluoroacétate d'alkyle linéaire, la réaction ayant lieu de préférence dans un alcool contenant 1 à 5 atomes de carbone et encore plus préférentiellement dans l'alcool ayant servi à préparer le trifluoroacétate d'alkyle. La réaction peut aussi avoir lieu dans un excès de trifluoroacétate d'alkyle. La lysine N, ε trifluoroacétylée est aisément séparée du milieu réactionnel et purifiée de manière connue en soi par tout procédé par exemple par recristallisation.

Actuellement la seule manière d'obtenir le dipeptide, de la sous forme pure est de le préparer sous une forme doublement protégée par un groupe benzyloxycarboxyle sur la fonction amine situé en α de la lysine et trifluoroacétyle sur la fonction aminée située en ε. Ce dipeptide protégé est purifié car il cristallise facilement puis par hydrogénolyse il conduit au dipeptide recherché sous forme pure et à des produits secondaires aisément distillables. L'inconvénient de cette méthode est essentiellement son coût dû à des frais de matière première et à l'étape supplémentaire de protection et déprotection de la fonction amine située en α.

La seule méthode économiquement envisageable de préparation du dipeptide à partir de la lysine trifluoroacétylée consiste à utiliser le phosgène qui est un réactif peu coûteux, qui permet de protéger la fonction amine située en α, qui active la fonction carboxyle et permet d'obtenir des rendements de condensation peptidique de l'ordre de 95 à 98 % en dipeptide.

La lysine N, ε trifluoroacétylée est condensée avec le phosgène par un procédé décrit par exemple par SELA, ARNON, JACOBSON dans Biopolymers, vol. 1, 517-525 (1963). Selon ce procédé on met en contact la lysine N, ε trifluoroacétylée avec du phosgène dans un solvant aprotique polaire à une température d'environ 50°C pour former l'anhydride de la N, ε trifluoroacétylée avec du phosgène dans un solvant aprotique polaire à une température d'environ 50°C pour former l'anhydride de la N, ε trifluoroacétyl- N, α carboxy-L-Lysine.

La proline sous sa forme sodée est mise en émulsion dans un mélange acétonitrile-eau puis mise en contact avec l'anhydride de la N, ε trifluoroacétyl-N, α carboxy-L lysine selon le procédé décrit par KATAKAI dans J. Org. Chem., vol. 37, n°2, p. 327-329 (1972).

En fin de réaction on obtient, comme décrit dans Large Scale Ncarboxy anhydride preparation of ala-Pro and ε-(TFA)-lys-pro. Synthesis of ACE inhibitors, Peptides funct., Proc. am.pept. Symp. 9th, 1985, 787-790, le dipeptide lysine trifluoroacétylée-proline en solution aqueuse mélangé à des polypeptides dont la majorité est constituées majoritairement de tripeptide lysine (TFA)-lysine(TFA)-proline, à de la proline libre, à des sels notamment du sulfate de sodium, du chlorure de sodium, et à des traces de lysine (TFA).

Cette solution contient le dipeptide à une concentration comprise entre 2 et 10 % en poids.

Le dipeptide est un composé extrêmement fragile qui craint
- les PH alcalins car il y a alors hydrolyse du groupe trifluoroacétamide,
- l'élévation de température qui accélère la formation de la dicétopipérazine par cyclisation interne.

L'obtention du dipeptide à l'état pur à partir de solutions brutes est un problème difficile qui n'a pas été résolu à ce jour car les sous produits formés lors de sa préparation ont des propriétés très voisines.

La présente invention a permis de résoudre les problèmes laissés dans l'art antérieur en ce qui concerne l'extraction du dipeptide sous forme pure de son milieu réactionnel.

Elle consiste à extraire le dipeptide ε trifluoroacétyl-lysyl-proline d'une solution aqueuse saturée en sel de préparation dudit peptide par extraction sélective à contre courant du dipeptide par un alcool primaire ou un mélange alcool primaire-solvant peu polaire.

La solution saturée en sel est une solution saturée par exemple en chlorure de sodium, chlorure de potassium, sulfate de sodium. L'alcool primaire est choisi avantageusement parmi les alcools primaires contenant de préférence 3 à 6 atomes de carbone.

On peut citer non limitativement
-l'alcool néopentylique,
- l'hexanol-1,
- le méthyl-2 propanol-1,
- le méthyl-2 butanol-1,
- le pentanol-2
- le butanol.
On préfère utiliser le butanol-1.

Le solvant aprotique est choisi parmi notamment
- l'acétate d;éthyle,
- l'acétonitrile,
- les éthers,
- le toluène,
- les alcanes.

L'extraction sélective des polypeptides se fait de préférence à un PH compris entre 4 et 7 et de préférence à un PH d'environ 5. L'extraction du dipeptide à partir d'une solution appauvrie en polypeptide ou l'extraction simultanée des di et polypeptides se fait notamment à un PH compris entre 1 et 4 et de préférence compris entre 1 et 3.

Le rapport volumique du solvant peu polaire à l'alcool est de préférence compris entre 0, lorsque l'on utilise de l'alcool pur, et 80 %. Il est encore plus préférentiellement d'environ 50 % en ce qui concerne l'acétate d'éthyle et le butanol .

L'extraction peut se faire indifféremment dans un sens ou dans l'autre c'est-à-dire d'abord extraction des polypeptides puis du dipeptide sans entraîner les acides aminés libres essentiellement la proline ou d'abord séparation de la proline puis extraction des polypeptides et du dipeptide.

Ainsi selon un premier procédé de mise en oeuvre quand on commence par éliminer les polypeptides et que l'on utilise comme alcool le butanol il est avantageux de pratiquer une série d'extractions successives de la solution aqueuse contenant le dipeptide et saturée en chlorure de sodium par le butanol ou une solution butanol-acétate d'éthyle à un PH compris entre 4 et 7 et de préférence à un PH d'environ 5. Le rapport volumique de la solution butanolique extractante à la solution aqueuse extraite est avantageusement compris entre 0,02 et 1. L'extrait butanolique contient en fonction du nombre d'extractions pratiquées au moins 90 % des polypeptides présents dans la phase aqueuse.

Dans une deuxième étape on récupère le dipeptide en phase butanolique par une extraction de la phase aqueuse à PH acide compris entre 1 et 4 et de préférence entre 1 et 3. Avantageusement le rapport volumique de la phase butanolique à la phase aqueuse est de un lorsque l'on pratique une seule extraction ou peut être seulement de 0,02 à 0,5 lorsque l'on pratique plusieurs extractions successives. Les aminoacides isolés tels que la proline sont laissés dans la phase aqueuse qui est rejetée. Le taux d'extraction du dipeptide atteint avantageusement 95 %. Cette solution est neutralisée par addition d'une solution aqueuse de bicarbonate.

Dans une troisième étape on effectue une distillation sous vide à une température inférieure à 30°C, les sels précipitent, ces sels sont filtrés et laissent subsister une solution de dipeptide dans le butanol.

Cette solution peut être utilisée telle quelle ou le dipeptide peut être isolé par précipitation à l'aide d'un alcane linéaire ou ramifié tel que notamment l'heptane, l'isooctane, le pentane, le méthylcyclohexane, d'un éther tel que le méthyltertiobutyléther, le diisopropyléther, d'un ester tel que l'acétate d'éthyle, d'une cétone telle que la méthylisobutylcétone.

Selon un second procédé de mise en oeuvre on élimine d'abord les aminoacides isolés avant d'éliminer les polypeptides.

Dans ce cas on extrait l'ensemble dipeptide et polypeptide par une solution butanolique à un PH comprise entre 1 et 7 et de préférence compris entre 1 et 3.

Le rapport du volume de la solution extractante butanolique à la solution aqueuse saline extraite est avantageusement compris entre 1 et 10. Les acides aminés libres tels que la proline notamment restent dans la solution aqueuse qui est rejetée.

Dans une deuxième étape on extrait la solution butanolique contenant le dipeptide et les polypeptides par une solution aqueuse acide à un PH compris entre 4 et 7 et de préférence d'environ 5. Le volume de la solution extractante à la solution butanolique est avantageusement compris entre 1 et 30. Le dipeptide reste en solution aqueuse alors que le tripeptide passe avantageusement en solution butanolique. Cette étape est la même que celle correspondant à l'étape 1 du 1er procédé de mise en oeuvre.

Le taux d'extraction des polypeptides est d'environ 90 à 95 %.

Dans une troisième étape on extrait le dipeptide de la solution aqueuse par du butanol à un PH avantageusement compris entre 1 et 3. Cette étape est identique à l'étape 2 du 1er procédé de mise en oeuvre.

Le dipeptide est récupéré par précipitation à l'aide d'un alcane.

La présente invention permet, d'obtenir un dipeptide présentant un taux de pureté supérieur à 96 % sur sec.

La présente invention sera plus complètement décrite à l'aide des exemples suivants qui ne doivent en aucun cas être considérés comme limitatifs.

## EXEMPLE 1

Solution aqueuse à extraire

| | |
|---|---|
| - dipeptide (ε lys TFA Pro) | = 4,53 % en poids (53,5 moles) |
| - tripeptide (ε lys TFA ε lys TFA Pro) | = 0,34 % en poids (2,4 moles) |
| - Proline | = 0,196 % en poids (6,8 moles) |
| - dicétopiperazine | = 0,019 % en poids (0,24 moles) |
| - NaCl | = concentration saturante. |

Solution extractante
- 32 kg de N butanol (40 litres)
- 36 kg d'acétate d'éthyle (40 litres)
- 17 kg de solution aqueuse à 26,4 % de NaCl ce qui fournit une solution après mélange composée de
- 73 kg de phase butanolique.

### 1-a Elimination du Tripeptide

1-a α

Dans un réacteur vitrifié de 800 litres on introduit 400 kg de solution aqueuse saturée en NaCl ayant la composition ci-dessus rappelée.

La phase aqueuse contenant le dipeptide est extraite par 3 fois 22 kg de la solution extractante. La durée d'agitation est de 1/2 heure à chaque fois.

Après chaque extraction on ajuste le PH de la phase aqueuse à environ 4,5. On soutire 90 kg de phase organique qui contient 85,3 % du tripeptide contenu dans la phase aqueuse de départ.

La phase aqueuse contient
- 49,8 moles de dipeptide (soit 93 % du dipeptide introduit)
- 0,35 moles de tripeptide
- 5,8 moles de Proline
- 0,01 mole de dicétopiperazine.

1-a β

Solution aqueuse à extraire

- dipeptide (ε Lys TFA Pro) = 4,41 % en poids
- tripeptide (ε Lys TPA Pro) = 0,48 % en poids
- NaCl = concentration saturante
- Volume 160 ml soit = 184,1 g

Solution extractante
- Mélange de n butanol et d'acétate d'éthyle (50 vol/50 vol) saturé par une solution saturée de NaCl dans l'eau.

Elimination du Tripeptide à PH 4,5

Dans une ampoule à décanter de 250 ml on introduit 160 ml de solution à extraire et 10 ml de solution extractante. L'ensemble est agité vigoureusement durant 5 mn, puis laissé au repos 20 mn et enfin décanté.

La phase aqueuse est extraite à nouveau à 4 reprises par le même volume de phase organique.

En fin d'opération, les 5 phases organiques sont réunies. La phase aqueuse résiduelle pèse 168 g,

| elle renferme | 86 % de la quantité initiale de dipeptide |
| et | 1 % de la quantité initiale de tripeptide. |

La concentration de ce dernier est donc de 50 ppm tandis que le rapport pondéral TRIPEPTIDE/DIPEPTIDE est égal à : 0,11 %.

1-b Extraction du dipeptide

La phase aqueuse issue de la 1ère série d'extraction (1a α) est amenée à PH 2,5 par addition d'acide chlorhydrique concentré. Le chlorhydrate de dipeptide formé est extrait par 4 fois 38,5 kg de N butanol saturé par une solution aqueuse de NaCl.

Les phases organiques successives sont soutirées et rassemblées, on obtient 196 kg de phase organique contenant 96,1 % du dipeptide contenu dans la phase aqueuse issue de la 1ère série d'extraction.

La phase aqueuse epuisée contient 1,9 moles de dipeptides et 6,6 moles de Proline.

1-c Neutralisation de la phase butanolique

La phase butanolique de 196 kg est neutralisée par 76 kg de bicarbonate de sodium en solution aqueuse à 5 % (PH 5,6).

Elle est ensuite concentrée sous pression réduite (40 mbar) à une température de 25°C dans le bouilleur et 20°C en tête de colonne pendant 20 heures.

On récupère 56 kg de concentré contenant moins de 0,02 % en poids d'eau mais contenant
- 43,55 moles de dipeptide
- 0,39 moles de tripeptide.

Après concentration les sels sont éliminés par filtration. On récupère en phase butanolique 89 % du dipeptide contenu dans la phase butanolique (rendement par rapport à la solution initiale à extraire = 79,5 %).

1-d α Précipitation du dipeptide à l'heptane

La phase butanolique contenant le dipeptide à une concentration de 27 % poids/poids (105 ml) . Cette solution a la composition suivante :

| - Lys (TFA)Pro | = | 96,5 % |
| - Pro | < | 0,1 % |
| - Lys (TFA) | < | 0,1 % |
| - Dicétopipera-zine | = | 0,85 % |
| - NaCl | = | 1,6 % |
| - Lys(TFA)Lys(-TFA)Pro | = | 0,8 % |

Cette solution conservée à - 14°C pendant deux mois laisse cristalliser une partie du dipeptide qu'elle contient.

Le solide est dissous en portant la suspension à 40°C durant 39 minutes. La solution homogène obtenue a toujours la même composition, le taux de dicétopiperazine n'a pas évolué. Si une partie de la solution initiale est conservée à 20°C au lieu de - 14°C, le taux de dicétopiperazine évolue de 0,8 à 6,3 % (P/P).

La solution n'ayant pas évolué est diluée par addition de 52,5 ml de n heptane.

La solution résultante est alors coulée en 1 heure dans un réacteur contenant 640 ml de n heptane, sous agitation vigoureuse.

Il se forme une suspension que l'on agite encore 30 mn après la fin de la coulée.

Le solide est recueilli par filtration sur un fritté n° 3.

Il est remis en suspension dans 70 ml d'une solution d'acétate d'éthyle à 10 % (en volume) dans le n heptane, essoré à nouveau, puis lavé à deux reprises par 70 ml de n heptane.

Les jus mères renferment 1,5 % du dipeptide introduit.

Le solide séché sous vide est obtenu avec un rendement global de 78,3 %. Sa composition est la suivante :

| | | |
|---|---|---|
| - Lys(TFA)Pro | = | 82,3 % |
| - H₂O | = | 1,7 % |
| - Acétate d'éthyle | = | 0,2 % |
| - n heptane | = | 5,7 % |
| - NaCl | = | 2,1 % |
| - Dicétopipera-zine | = | 3,9 % |
| - Tripeptide | = | 0,9 % |
| - n Butanol | = | 5,7 % |
| | | 102,3 % |

1-d β Précipitation du dipeptide à l'ether isopropylique

Un aliquot de la solution (à 27 % de dipeptide poids pour poids) est conservé à +4°C / +20°C durant 75 jours.

Sa composition est alors la suivante :
- Lys(TFA)Pro = 24,5 % poids/poids solution butanolique
- Dicétopiperazine/Lys(TFA)Pro = 10,8 % poids/poids

Dans un réacteur en pyrex d'une capacité de 1 litre à 22°C, sont introduits successivement:
- 700 ml de diisopropyl oxyde
- sous agitation (200 tours/minute) 105 ml (97,18 g) de solution butanolique, coulée régulièrement en 1 heure.

La suspension de dipeptide est agitée encore 15 minutes puis elle est soutirée par la vanne de fond et transférée sur un verre fritté de porosité n° 3 et de diamètre égal à 100 mm. L'essorage est rapide.

Le solide est remis en suspension à trois reprises avec 75 ml de diisopropyloxyde, essoré à fond puis étuvé à température ambiante sous 0,2 torr.

RESULTATS : Rendement de la précipitation : 98 %

Composition du solide

| | | |
|---|---|---|
| - Lys(TFA)Pro | = | 84,0 % p/p |
| - Dicétopipéra-zine | = | 4,5 % p/p |
| - Tripeptide | = | 0,8 % p/p |
| - n Butanol | = | 1,7 % p/p |
| - Disopropylé-ther | = | 5,3 % p/p |
| - Eau | = | 1,5 % p/p |
| - NaCl | = | 2,0 % p/p |

EXEMPLE 2

Cet exemple sert à démontrer la faisabilité du 2ème moyen de mise en oeuvre de l'invention ; il n'a pas été optimisé quant aux pH d'extraction.

Solution aqueuse à extraire :

| | | |
|---|---|---|
| - dipeptide (ε Lys TFA Pro) | = | 4,95 % poids/poids (73 mmoles) |
| - tripeptide (ε Lys TFA ε Lys TFA Pro) | = | 0,18 % poids/poids (1,6 mmoles) |
| - proline | = | 0,32 % poids/poids (14 mmoles) |
| - dicétopipéra-zine | < | 0,01 % poids/poids |
| - NaCl | = | concentration saturante |
| - pH | = | 5,5 |

Solution extractante :

| | | |
|---|---|---|
| - N butanol | = | 5 litres |
| - Solution aqueuse à 26,4 % de NaCl | = | 1 kg |

2-a Extraction des peptides

Dans un réacteur en pyrex d'une capacité de 1 litre on introduit 500 g de solution aqueuse à extraire ayant la composition ci-dessus mentionnée.

Cette phase aqueuse est extraite à 10 reprises par 0,5 litre de la solution extractante. La durée de l'agitation est de 3 minutes à chaque fois, tandis que la durée de la décantation est de 15 minutes.

La phase butanolique pèse au total 4 420 g et renferme :
- 67,5 mmoles de dipeptide (92 % du dipeptide introduit)
- 1,54 mmoles de tripeptide
- 0,014 mmoles de proline.

2-b Concentration de la phase butanolique

La phase butanolique est concentrée par évaporation du butanol sous vide (rendement quantitatif). Après filtration du précipité de NaCl on obtient une solution de dipeptide à 20,4 % en poids dans le butanol.

2-c Elimination du tripeptide

Une partie de 64 g de solution organique obtenue ci-avant par concentration, renfermant :
- 13 g de dipeptide (38 mmoles)
- 0,93 g de tripeptide (1,65 mmoles)
est extraite par 6 x 75 ml et 9 x 150 ml de saumure saturée à pH3.

Les extraits aqueux rassemblés, d'une masse totale de 2 420 g, contiennent :
- 12,5 g de dipeptide (soit 96 % de la quantité introduite)
- 0,08 g de tripeptide.

## 2-d Concentration

La phase aqueuse obtenue ci-avant est concentrée, à 30°C par distillation sous vide, jusqu'à une teneur de 5 % en dipeptide.

## 2-e Isolement du dipeptide

Il est réalisé selon l'exemple 3 : opérations 3d à 3g incluses.

## EXEMPLE 3

Solution aqueuse à extraire :

| | |
|---|---|
| - dipeptide | 5,75 % en poids |
| - tripeptide | 0,36 % en poids |
| - proline | 0,60 % en poids |
| - NaCl | concentration saturante |
| - pH | 5,5. |

Solution extractante :
- n butanol saturé par contact avec une solution de saumure.

## 3-a Extraction des peptides

L'extraction est réalisée à contre-courant au moyen d'une colonne agitée de marque KUHNI, d'un diamètre de 60 mm, comprenant 40 compartiments agités à 190 tours par minute (hauteur totale 3,88 m).

La phase aqueuse est injectée au sommet de cette colonne, tandis que la phase butanolique est injectée à la base à un débit 2,65 fois supérieur.

On obtient un extrait organique renfermant 86,4 % du dipeptide introduit à une concentration de 1,8 % en poids. La concentration du tripeptide est alors de 0,15 %.

## 3-b Concentration de l'extrait butanolique

L'extrait butanolique est concentré à 30°C sous vide. Le chlorure de sodium qui précipite est éliminé par filtration on obtient alors une solution renfermant 86,4 % du dipeptide introduit initialement sous forme de solution aqueuse à extraire.

## 3-c Elimination du tripeptide

La même colonne agitée est utilisée pour éliminer le tripeptide.

La solution butanolique précédente à 3,68 % de dipeptide est injectée à la base de la colonne, tandis que l'on introduit au sommet une solution saturée en NaCl de pH 4,4 avec un débit 2,3 fois supérieur.

A la base de la colonne on recueille la phase aqueuse contenant 86,4 % du dipeptide introduit initialement à l'étage 30. Cette solution, d'une concentration en dipeptide de 1,6 %, contient seulement 0,029 % de tripeptide en poids.

Le tripeptide est retrouvé en totalité dans l'effluent butanolique de concentration en tripeptide égale à 0,29 % en poids.

## 3-d Extraction du dipeptide en milieu organique

La solution aqueuse saturée en NaCl précédemment obtenue est concentrée sous vide de 1 torr à 25°C.

A partir de 3,357 kg de cette solution à 1,6 % en dipeptide on obtient après filtration du précipité de chlorure de sodium 0,910 kg de phase aqueuse à 5,9 % poids/poids de dipeptide soit 53,69 g de dipeptide.

Cette phase aqueuse est extraite par 15 fois 450 ml d'une solution d'acétate d'éthyle à 20 % (volume/volume) dans le butanol.

Les extraits organiques contiennent 44 g de dipeptide soit un rendement d'extraction de 81,8 % ce qui correspond à 70,7 % du dipeptide introduit.

## 3-e Concentration de l'extrait butanolique

La solution précédente pesant 8 440 g et renfermant 44 g de dipeptide est concentrée sous 0,5 Torr à 25°C. On élimine l'azéotrope butanol-eau et on observe la formation d'un précipité de chlorure de sodium que l'on enlève par filtration.

La solution concentrée obtenue, d'une masse de 192 g contient 44 g de dipeptide (concentration 23 %) et 0,3 g de NaCl.

## 3-f Précipitation du dipeptide par un non-solvant

La phase butanolique concentrée est coulée dans un réacteur agité contenant 1 litre d'heptane à 23°C ce qui génère un précipité.

Le précipité est filtré puis séché jusqu'à un poids constant de 45,4 g. Le produit est sous forme de poudre.

Une partie de 38 g de cette poudre est remise en suspension dans un mélange composé de 450 ml d'heptane et 50 ml d'acétate d'éthyle.

Après 2 heures d'agitation le solide est isolé par filtration sur verre fritté de porosité n°4. Il est lavé par 2 x 100 ml du mélange heptane-acétate d'éthyle (90-10) et séché sous 1 Torr pendant 6 heures à température ambiante.

Le poids du solide obtenu est de 36,78 g, son titre de 92 %. Le rendement de précipitation est de 91,8 %.

Le rendement global à partir de la solution aqueuse à extraire est de 64,9 %.

Méthode

| | | |
|---|---|---|
| - dipeptide | 92,0 % en poids | CLHP |
| - tripeptide | 1,8 % en poids | " |
| - dicétopipérazine | 1,0 % en poids | " |
| - proline | non décelé | " |
| - NaCl | 1,76 | dosage C1 argentimétrique |
| - H$_2$O | 2,4 | |
| - butanol | 2 | chromatographie gazeuse |
| - heptane | 0,54 | |
| - acétate d'éthyle | 0,52 | |

. Dosage de l'acidité par Bu$_4$N$^+$OH-

1er saut (COOH) : 2,72 équivalent/kg (théorie 2,95)

2ème saut (NHCOCF$_3$) : 2,72 équivalent/kg

soit un titre potentiométrique de 92,2 %.

. $[\alpha]_D^{21} = -36,5°$ (solution à 1 % dans EtOH).

. Point de fusion 105°C.

. Identité confirmée par spectrométrie de Masse et spectrométrie de RMN[1]H et $^{13}$C à 1 et 2 dimensions (360 MHz, solvant CH$_3$OD).

**Revendications**

1 - Procédé de purification et d'extraction du dipeptide ε trifluoroacétyl-lysyl-proline à partir d'une solution aqueuse saturée en sel contenant le dipeptide, des polypeptides et des acides aminés isolés caractérisé en ce qu'on sépare le dipeptide des autres composés par une série d'extractions successives par un alcool primaire ou un mélange alcool primaire-solvant aprotique.

2 - Procédé selon la revendication 1 caractérisé en ce que la solution d'extraction est composée d'un alcool primaire choisi parmi les alcools primaires contenant de préférence 3 à 6 atomes de carbone.

3 - Procédé selon la revendication 2 caractérisé en ce que l'alcool est choisi parmi le butanol, l'alcool néopentylique, l'hexanol, le méthyl 2 propanol, le butanol, le pentanol.

4 - Procécé selon la revendication 1 caractérisé en ce que le solvant aprotique est choisi de préférence parmi l'acétate d'éthyle, l'acétonitrile, les éthers, le toluène et les alcanes contenant 6 à 8 atomes de carbone.

5 - Procédé selon les revendications 2 à 4 caractérisé en ce que le solvant d'extraction est un mélange butanol-acétate d'éthyle contenant 20 à 100 % en volume de butanol et de préférence 50 %.

6 - Procédé selon la revendication 1 caractérisé en ce que l'extraction sélective des polypeptides est réalisée à un PH compris entre 4 et 7 et de préférence à un PH égal à environ 5.

7 - Procédé selon les revendications 1 et 6 caractérisé en ce que l'extraction du dipeptide est réalisée à un PH compris entre 1 et 4 et de préférence compris entre 1 et 3.

8 - Procédé de précipitation du dipeptide ε trifluoroacyl-lys-proline à partir de la solution butanolique caractérisé en ce que on ajoute à ladite solution un solvant choisi parmi les alcanes, les éthers, les cétones, les esters et de préférence parmi les éthers.

9 - Procédé de purification et d'extraction du dipeptide ε trifluoroacétyl-lysyl-proline à partir d'une solution aqueuse saline contenant le dipeptide, des polypeptides et éventuellement des amino acides et des sels minéraux caractérisé en ce que dans un 1ère étape on fait une série d'extractions successives par un solvant à base de butanol à un PH compris entre 4 et 7 et de préférence égal à environ 5 de façon à extraire sélectivement les polypeptides, dans une 2ème étape on extrait la solution aqueuse résiduaire de la 1ère étape par une solution butanolique à un PH compris entre 1 et 4 et de préférence entre 1 et 3 et on élimine la phase aqueuse résiduaire contenant la proline, dans un 3ème étape on neutralise la solution butanolique par une solution alcaline aqueuse et on effectue une distillation sous pression réduite

dans une 4ème étape éventuelle on précipite le dipeptide par ajout d'un alcane ou d'un éther.

10 - Procédé de purification et d'extraction du dipeptide ε trifluoroacétyl-lysyl-proline à partir d'une solution aqueuse contenant, le dipeptide, des polypeptides et éventuellement des aminoacides et des sels minéraux caractérisé en ce que dans une 1ère étape on fait une extraction par un solvant à base de butanol à un PH compris entre 1 et 4 et de préférence entre 1 et 3 on élimine une solution aqueuse contenant la proline,

dans une 2ème étape on extrait le dipeptide et les sels par une solution aqueuse acide à un PH compris entre 4 et 7 de préférence égal à 5 et on élimine les polypeptides en solution organique,

dans une 3ème étape on extrait sélectivement le dipeptide par une solution butanolique dans une 4ème étape éventuelle on précipite le dipeptide à partir de la solution butanolique par un alcane ou un éther.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | JOURNAL OF ORGANIC CHEMISTRY, vol. 53, 1988, pages 836-844, American Chemical Society; T.J. BLACKLOCK et al.: "Synthesis of semisynthetic dipeptides using N-carboxyanhydrides and chiral induction on Raney nickel. A method practical for large scale" * En entier, en particulier page 843, colonne de gauche, paragraphe 1 * --- | 1-10 | C 07 K  1/14<br>C 07 K  5/00<br>C 07 K  5/06 |
| A,D | PEPTIDES, STRUCTURE AND FUNCTION, pages 787-790, Pierce Chemical Co., Rockford, Illinois, US; T.J. BLACKLOCK et al.: "Large scale N-carboxyanhydride preparation of ALA-PRO and Nepsilon-(TFA)- LYS-PRO: synthesis of ace inhibitors" * En entier * --- | 1-10 | |
| A,D | EP-A-0 168 769  (MERCK AND CO.) * Page 11 * --- | 1-10 | |
| A | FR-A-2 591 226  (AJINOMOTO) * En entier * ----- | 1 | |

DOMAINES TECHNIQUES RECHERCHES (Int. Cl.4)

C 07 K  1/00
C 07 K  5/00

Le présent rapport a été établi pour toutes les revendications

| Lieu de la recherche | Date d'achèvement de la recherche | Examinateur |
|---|---|---|
| LA HAYE | 10-08-1989 | MASTURZO P. |